# EUROPEAN PATENT APPLICATION

(11) **EP 1 862 461 A1**
(43) Date of publication of application: **05.12.2007**
(21) Application number: 06729454.6
(22) Date of filing: 20.03.2006
(51) Int. Cl.: C07D 237/12

(54) **METHOD FOR PRODUCING 3,6-DICHLOROPYRIDAZINE-1-OXIDE**

(30) Priority: 22.03.2005 JP 2005082174; 22.03.2005 JP 2005082175
(71) Applicant: Sankyo Agro Company, Limited, Tokyo 105-7117 (JP)
(72) Inventor: TSUKAMOTO, Yoshihisa c/o Sankyo Agro Company, Limited, Tokyo 105-7117 (JP); KUDO, Noriaki c/o Sankyo Agro Company, Limited, Tokyo 105-7117 (JP); KANEKO, Toshio c/o Sankyo Agro Company, Limited, Tokyo 105-7117 (JP); KOMAI, Hiroyuki c/o Sankyo Agro Company, Limited, Tokyo 105-7117 (JP); NAKAGAWA, Harumi c/o Sankyo Agro Company, Limited, Tokyo 105-7117 (JP)
(74) Representative: Srinivasan, Ravi Chandran
(86) International application number: PCT/JP2006/305486
(87) International publication number: WO 2006/101058

(57) **Abstract**

A process for preparing 3,6-dichloropyridazine-1-oxide which comprises reacting 3,6-dichloropyridazine with an acid anhydride and hydrogen peroxide of a concentration of 60% or less or a urea hydrogen peroxide addition compound.

## Description

### TECHNICAL FIELD

The present invention relates to a novel and simple process for preparing 3,6-dichloropyridazine-1-oxide useful as a synthesis intermediate of a physiologically active substance.

### BACKGROUND ART

In Non-patent document 1, a process in which 3,6-dichloropyridazine is reacted with monoperphthalic acid in ether to prepare 3,6-dichloropyridazine-1-oxide is described. In this process, however, the yield of 3,6-dichloropyridazine-1-oxide is 9.4% or less and the process is unsatisfactory as a preparation process.

In Non-patent document 2, a process in which 3,6-dichloropyridazine is reacted with perbenzoic acid in chloroform to prepare 3,6-dichloropyridazine-1-oxide is described. In this process, however, the reaction time is a long period of time as long as two weeks, and further the yield of 3,6-dichloropyridazine-1-oxide is 50% or less, thus the process being unsatisfactory as a preparation process.

In Non-patent document 3, a process in which 3,6-dichloropyridazine is reacted with 74% hydrogen peroxide and maleic anhydride in dichloromethane to prepare 3,6-dichloropyridazine-1-oxide is described. In this process, however, the reaction time is a long period of time as long as 7 days, the yield of 3,6-dichloropyridazine-1-oxide is 50% and further 74% hydrogen peroxide is hardly available, thus the process being unsatisfactory as a preparation process.

In Non-patent document 4, a process in which 3,6-dichloropyridazine is reacted with 90% hydrogen peroxide and dichloromaleic anhydride in dichloromethane to prepare 3,6-dichloropyridazine-1-oxide is described. In this process, 3,6-dichloropyridazine-1-oxide is obtained at yield of 86%. However, 90% hydrogen peroxide is hardly available and its handling is difficult, thus the process being unsatisfactory as a preparation process.
Non-patent document 1: Chemical and Pharmaceutical Bulletin, 1962, vol. 10, No. 10, 989-992
Non-patent document 2: Journal of the Pharmaceutical Society of Japan, 1962, vol. 82, No. 2, 244-248
Non-patent document 3: Journal of Heterocyclic Chemistry, vol. 9, 1972, 351-354
Non-patent document 4: Synthesis, 1973, 495-496

### DISCLOSURE OF INVENTION

### PROBLEM TO BE SOLVED BY THE INVENTION

Accordingly, it is an object of the present invention to provide a simple synthesis method with a high yield in view of importance of 3,6-dichloropyridazine-1-oxide as a synthesis intermediate of the physiologically active substance.

### MEANS FOR SOLVING THE PROBLEM

The present inventors intensively studied in order to accomplish the above object, and, as a result, have found that 3,6-dichloropyridazine-1-oxide can be obtained at high yield by reacting 3,6-dichloropyridazine with hydrogen peroxide of a concentration of 60% or less or a hydrogen peroxide addition compound of urea (Urea hydrogen peroxide addition compound, UHP) in the presence of an appropriate acid anhydride to complete the present invention.

Namely, the present invention is:
(1) A process for preparing 3,6-dichloropyridazine-1-oxide which comprises reacting 3,6-dichloropyridazine with an acid anhydride and hydrogen peroxide of a concentration of 60% or less or a urea hydrogen peroxide addition compound.
(2) The preparation process according to (1), wherein 3,6-dichloropyridazine is reacted with the acid anhydride and the hydrogen peroxide of a concentration of 60% or less.
(3) The preparation process according to (1), wherein 3,6-dichloropyridazine is reacted with the acid anhydride and the urea hydrogen peroxide addition compound.
(4) The preparation process according to any one of (1) to (3), wherein the acid anhydride is one kind or more of acid anhydrides selected from acetic/formic anhydride, acetic anhydride, trichloroacetic anhydride, trifluoroacetic anhydride, propionic anhydride, butyric anhydride, succinic anhydride, maleic anhydride, dichloromaleic anhydride, phthalic anhydride, 3,6-dichlorophthalic anhydride, tetrachlorophthalic anhydride and tetrabromophthalic anhydride.
(5) The preparation process according to (4), wherein the acid anhydride is one kind or more of acid anhydrides selected from trifluoroacetic anhydride, maleic anhydride, dichloromaleic anhydride, 3,6-dichlorophthalic anhydride, tetrachlorophthalic anhydride and tetrabromophthalic anhydride.
(6) The preparation process according to (5), wherein the acid anhydride is maleic anhydride.
(7) The preparation process according to (5), wherein the acid anhydride is dichloromaleic anhydride.
(8) The preparation process according to (5), wherein the acid anhydride is trifluoroacetic anhydride.
(9) The preparation process according to (5), wherein the acid anhydride is tetrachlorophthalic anhydride.
(10) The preparation process according to any one of (1) to (9), wherein an acid is added to the reaction system.
(11) The preparation process according to (10), wherein the acid is formic acid, acetic acid, propionic acid, butyric acid, isobutyric acid, pivalic acid, cyclohexanecarboxylic acid, 1-methylcyclohexanecarboxylic acid, 1-adamantanecarboxylic acid, acrylic acid, methacrylic acid, crotonic acid, 3,3-dimethylacrylic acid, tiglic acid, cinnamic acid, trifluoroacetic acid, chloroacetic acid, benzoic acid, methanesulfonic acid, trifluoromethanesulfonic acid or p-toluenesulfonic acid.
(12) The preparation process according to (11), wherein the acid is formic acid, acetic acid, propionic acid, butyric acid, isobutyric acid, pivalic acid, cyclohexanecarboxylic acid, 1-methylcyclohexanecarboxylic acid, 1-adamantanecarboxylic acid, acrylic acid, methacrylic acid, crotonic acid, 3,3-dimethylacrylic acid, tiglic acid, cinnamic acid, trifluoroacetic acid or chloroacetic acid.
(13) The preparation process according to any one of (1) to (12), wherein a dehydrating agent is added to the reaction system.
(14) The preparation process according to (13), wherein the dehydrating agent is anhydrous magnesium sulfate.
(15) The preparation process according to any one of (1) to (14), wherein the hydrogen peroxide is added in an amount of 1 to 5 equivalents and the acid anhydride is added in an amount of excessive mole number relative to the mole number of water existing in the reaction system, or the urea hydrogen peroxide addition compound is added in an amount of 1 to 5 equivalents, with respect to 3,6-dichloropyridazine.

### EFFECTS OF THE INVENTION

According to the process of the present invention, 3,6-dichloropyridazine-1-oxide, i.e., a synthesis intermediate of a physiologically active substance can be synthesized more easily at high yield.

### BEST MODE FOR CARRYING OUT THE INVENTION

As the 3,6-dichloropyridazine, i.e., the starting material of the process of the present invention, a substance commercially available is used, or the 3,6-dichloropyridazine can be prepared by chlorinating, with phosphorus oxychloride, 1,2-dihydropyridazine-3,6-dione obtained from maleic anhydride and hydrazine according to the method described in Helvetica Chimica Acta, vol. 85, 2195-2213 (2002). Further, 3,6-dichloropyridazine can be also prepared according to the method described in Journal of the American Chemical Society, vol. 73, 1873-1874 (1951), USP 2671086 specification or the like.

The acid anhydride used in the process of the present invention is not particularly limited so long as it is reacted with hydrogen peroxide to give a peroxy acid. The acid anhydride employable can include an anhydride of a lower alkylcarboxylic acid, an anhydride of a lower halo-alkylcarboxylic acid, an anhydride of a lower alkenylcarboxylic acid, an anhydride of a lower halo-alkenylcarboxylic acid, an anhydride of a lower alkynylcarboxylic acid, an anhydride of an aromatic carboxylic acid, an anhydride of a halogenated aromatic carboxylic acid, an anhydride of a lower alkyldicarboxylic acid, an anhydride of a lower halo-alkyldicarboxylic acid, an anhydride of a lower alkenyldicarboxylic acid, an anhydride of a lower halo-alkenyldicarboxylic acid, an anhydride of an aromatic dicarboxylic acid, an anhydride of a halogenated aromatic dicarboxylic acid, an anhydride of a lower alkylsulfonic acid, an anhydride of a lower halo-alkylsulfonic acid, an anhydride of a lower alkenylsulfonic acid, an anhydride of a lower halo-alkenylsulfonic acid, an anhydride of a lower alkynylsulfonic acid, an anhydride of an aromatic sulfonic acid, an anhydride of a halogenated aromatic sulfonic acid, an anhydride of a lower alkyldisulfonic acid, an anhydride of a lower halo-alkyldisulfonic acid, an anhydride of a lower alkenyldisulfonic acid, an anhydride of a lower halo-alkenyldisulfonic acid, an anhydride of an aromatic disulfonic acid, an anhydride of a halogenated aromatic disulfonic acid and a mixed acid anhydride of these acids, preferably acetic/formic anhydride, acetic anhydride, trichloroacetic anhydride, trifluoroacetic anhydride, propionic anhydride, butyric anhydride, succinic anhydride, maleic anhydride, dichloromaleic anhydride, phthalic anhydride, 3,6-dichlorophthalic anhydride, tetrachlorophthalic anhydride and tetrabromophthalic anhydride, more preferably trifluoroacetic anhydride, maleic anhydride, dichloromaleic anhydride, 3,6-dichlorophthalic anhydride, tetrachlorophthalic anhydride and tetrabromophthalic anhydride.

In cases where hydrogen peroxide of a concentration of 60% or less is used, the amount of the acid anhydride used in the present invention is usually 1 to 20 mol relative to 1 mol of 3,6-dichloropyridazine, preferably 2 to 10 mol. When 3,6-dichloropyridazine and hydrogen peroxide are reacted, 3,6-dichloropyridazine-1-oxide can be obtained at higher yield by using the acid anhydride in an excessive amount relative to the mole number of water existing in the reaction system. Further, as described later, 3,6-dichloropyridazine-1-oxide can be obtained at higher yield by using the acid anhydride in an excessive amount relative to the mole number of water remaining in the reaction system after water in the reaction system is removed to some extent by dehydration with a dehydrating agent or the like. Further, in cases where the acid anhydride can be regenerated from an acid (maleic acid, dichloromaleic acid, 3,6-dichlorophthalic acid, tetrachlorophthalic acid, tetrabromophthalic acid or the like) corresponding to the acid anhydride such as maleic anhydride, dichloromaleic anhydride, 3,6-dichlorophthalic anhydride, tetrachlorophthalic anhydride, tetrabromophthalic anhydride or the like by an intramolecular dehydration reaction, 3,6-dichloropyridazine-1-oxide can be obtained at higher yield even if the acid anhydride of less mole number than the mole number of water existing in the reaction system is used.

In cases where the urea hydrogen peroxide addition compound is used, the amount of the acid anhydride used in the process of the present invention is usually 0.5 to 5 mol, preferably 1 to 3 mol, relative to 1 mol of 3,6-dichloropyridazine.

The hydrogen peroxide used in the present invention is hydrogen peroxide of a concentration of 60% or less and a commercially available one can be used. The concentration of the hydrogen peroxide is not particularly limited so long as it is 60% or less and is preferably 20 to 60%, more preferably 50 to 60%. The amount of hydrogen peroxide used is usually 0.5 to 5 mol (0.5 to 5 equivalents), preferably 1 to 5 mol (1 to 5 equivalents), and more preferably 1 to 3 mol (1 to 3 equivalents) relative to 1 mol of 3,6-dichloropyridazine.

As the urea hydrogen peroxide addition compound used in the process of the present invention, a commercially available one may be used, or it can be prepared according to a method described in Japanese Unexamined Patent Publication No. 2002-60380 or the like.

The amount of the urea hydrogen peroxide addition compound used in the present invention is usually 0.5 to 5 mol (0.5 to 5 equivalents), preferably 1 to 5 mol (1 to 5 equivalents), and more preferably 1 to 3 mol (1 to 3 equivalents) relative to 1 mol of 3,6-dichloropyridazine.

The process of the present invention can be carried out in the presence or absence of a solvent. The solvent employable is not particularly limited so long as it does not affect the reaction and can include hydrocarbons such as hexane, heptane, benzene, toluene and xylene; halogenated hydrocarbons such as dichloromethane, chloroform, 1,2-dichloroethane, carbon tetrachloride and chlorobenzene; nitriles such as acetonitrile; carboxylic acids such as formic acid, acetic acid and trifluoroacetic acid; and a mixture of these solvents. In cases where carboxylic acid is used as the solvent, the solvent can be also used as an acid described later.

The present invention is carried out, if necessary, using an acid. The amount of acid is usually 0.01 to 100 mol, preferably 0.1 to 2 mol relative to 1 mol of 3,6-dichloropyridazine in cases where the hydrogen peroxide of a concentration of 60% or less is used. In cases where the urea hydrogen peroxide addition compound is used, the amount of acid is usually 0.01 mol or more, preferably 0.1 to 20 mol relative to 1 mol of 3,6-dichloropyridazine. 3,6-Dichloropyridazine-1-oxide can be obtained at higher yield by adding an acid.

The acid employable can include carboxylic acids such as formic acid, acetic acid, propionic acid, butyric acid, isobutyric acid, pivalic acid, cyclohexanecarboxylic acid, 1-methylcyclohexanecarboxylic acid, 1-adamantanecarboxylic acid, acrylic acid, methacrylic acid, crotonic acid, 3,3-dimethylacrylic acid, tiglic acid, cinnamic acid, trifluoroacetic acid, chloroacetic acid and benzoic acid; and sulfonic acids such as methanesulfonic acid, trifluoromethanesulfonic acid or p-toluenesulfonic acid, preferably formic acid, acetic acid, propionic acid, butyric acid, isobutyric acid, pivalic acid, cyclohexanecarboxylic acid, 1-methylcyclohexanecarboxylic acid, 1-adamantanecarboxylic acid, acrylic acid, methacrylic acid, crotonic acid, 3,3-dimethylacrylic acid, tiglic acid, cinnamic acid, trifluoroacetic acid or chloroacetic acid.

The process of the present invention is carried out, if necessary, using a dehydrating agent. The dehydrating agent employable is not particularly limited so long as it does not affect the reaction and can include molecular sieves (3A), molecular sieves (4A), anhydrous magnesium sulfate and anhydrous sodium sulfate, preferably anhydrous magnesium sulfate.

In the present invention, it is particularly preferably in view of yield that the hydrogen peroxide is added at 1 to 5 equivalents relative to 3,6-dichloropyridazine and the acid anhydride is added in an excessive mole number relative to the mole number of water existing in the reaction system. Further, in the present invention, it is particularly preferably in view of yield that the urea hydrogen peroxide addition compound is added at 1 to 5 equivalents relative to 3,6-dichloropyridazine.

In the present invention, the reaction is usually carried out at a temperature ranging from -78 to 150°C, preferably from -10°C to 80°C.

In the present invention, in cases where hydrogen peroxide of a concentration of 60% is used, the reaction time is generally from 30 minutes to 5 days, preferably from 1 hour to 3 days. In the present invention, in cases where the urea hydrogen peroxide addition compound is used, the reaction time is generally from 30 minutes to 4 days, preferably from 1 hour to 3 days.

After completion of the reaction, the desired 3,6-dichloropyridazine-1-oxide can be collected from the reaction mixture according to a conventional method. For example, 3,6-dichloropyridazine-1-oxide can be obtained by pouring the reaction mixture into water, extracting it with a water-immiscible solvent, drying the extract liquid and distilling off the solvent. The 3,6-dichloropyridazine-1-oxide can be purified, if necessary, by a conventional method such as recrystallization and column chromatography.

### EXAMPLES

In the following, the process of the present invention will be explained in more detail by Examples, Reference example and Test example, but the present invention is not limited thereto.

### Example 1

0.61 ml (13.5 mmol) of 60% hydrogen peroxide and 0.26 ml (3.4 mmol) of trifluoroacetic acid were added to a mixture of 1.00 g (6.71 mmol) of 3,6-dichloropyridazine and 1,2-dichloroethane (9.7 mL) at room temperature. The mixture was cooled with ice and 1.02 g (6.11 mmol) of dichloromaleic anhydride was added thereto with stirring. The reaction mixture was cooled with ice and 1.00 g (5.99 mmol), 1.00 g (5.99 mmol), 1.03 g (6.17 mmol) and 1.02 g (6.11 mmol) of dichloromaleic anhydride were added thereto with stirring after 30 minutes, 60 minutes, 90 minutes and 120 minutes, respectively (the amount of water in the reaction system was 0.30 g (16.7 mmol)). Thereafter, the mixture was stirred at room temperature for 19 hours, and then the reaction mixture was cooled with ice and a 10% aqueous sodium sulfite solution (10 mL) was added thereto. The mixture was stirred at 0°C for 1 hour, followed by extraction with dichloromethane. The organic layer was washed with a 4% aqueous sodium hydroxide solution and the washing liquid was re-extracted with dichloromethane. This was combined with the organic layer and it was washed with a saturated aqueous ammonium chloride solution and aqueous NaCl solution in this order, followed by drying with anhydrous magnesium sulfate and concentrating under reduced pressure to obtain 816 mg of 3,6-dichloropyridazine-1-oxide (purity: 96.4%, yield: 71%).

### Example 2

0.61 ml (13.5 mmol) of 60% hydrogen peroxide and 441 mg (3.66 mmol) of anhydrous magnesium sulfate were added to 1,2-dichloroethane (10 mL) under ice-cooling (the amount of water in the reaction system was 0.30 g (16.7 mmol) but it was almost completely dehydrated by the dehydrating agent (anhydrous magnesium sulfate)). The mixture was stirred under ice-cooling for 30 minutes and 2.25 g (13.5 mmol) of dichloromaleic anhydride and 1.01 g (6.79 mmol) of 3,6-dichloropyridazine were added thereto. After the mixture was stirred at room temperature for 19 hours, the reaction mixture was ice-cooled and a 10% aqueous sodium sulfite solution (10 mL) was added thereto. After the mixture was stirred at 0°C for 1 hour, it was extracted with dichloromethane. The organic layer was washed with a 4% aqueous sodium hydroxide solution and the washing liquid was re-extracted with dichloromethane. This was combined with the organic layer and washed with a saturated aqueous ammonium chloride solution and aqueous NaCl solution in this order, followed by drying with anhydrous magnesium sulfate and concentrating under reduced pressure to obtain 0.890 g of 3,6-dichloropyridazin-1-oxide (purity: 89%, yield: 70.8%).

### Example 3

10.0 g (67.1 mmol) of 3,6-dichloropyridazine, 59.3 g (605 mmol) of maleic anhydride, 0.58 g (6.7 mmol) of crotonic acid and 1,2-dichloroethane (100 ml) were mixed, and the mixture was stirred for 40 minutes. 11.52 ml (201 mmol) of 50% hydrogen peroxide was added to the mixture with stirring and after the mixture was stirred at room temperature for 40 hours and 30 minutes, 1,2-dichloroethane (100 ml) was added thereto and the mixture was ice-cooled (the amount of water in the reaction system was 6.85 g (381 mmol)). An aqueous sodium hydroxide solution [prepared from 48.3 g (1.21 mol) of sodium hydroxide and 200 ml of water] was added thereto under ice-cooling over 1 hour and 30 minutes. 2.6 g (20.6 mmol) of sodium sulfite was added thereto and the mixture was stirred under ice-cooling for 1 hour. 50 ml of water was added thereto and the mixture was filtered. The filtrate was separated to obtain an organic layer A and an aqueous layer A. The aqueous layer A was extracted with 1,2-dichloroethane (3×50 ml) to obtain an organic layer B and an aqueous layer B. The organic layer A and the organic layer B were combined and the mixture was washed with water (100 ml) to obtain an organic layer C and an aqueous layer C. The aqueous layer C was extracted with 1,2-dichloroethane (2x50 ml) to obtain an organic layer D and an aqueous layer D. The organic layer C and the organic layer D were combined, dried with anhydrous sodium sulfate and concentrated under reduced pressure to obtain 9.39 g of 3,6-dichloropyridazine-1-oxide (purity: 97.9%, yield: 83.0%).

### Example 4

1.60 ml (13.9 mmol) of pivalic acid and 0.92 ml (20.2 mmol) of 60% hydrogen peroxide were added to a mixture of 1.00 g (6.71 mmol) of 3,6-dichloropyridazine, 4.63 g (47.2 mmol) of maleic anhydride and 1,2-dichloroethane (8.5 mL) at room temperature, and the mixture was stirred at room temperature for 5 days (the amount of water in the reaction system was 0.46 g (25.6 mmol)). The reaction mixture was ice-cooled and a 10% aqueous sodium sulfite solution (19 mL) was added thereto. After the mixture was stirred at 0°C for 1 hour, the mixture was filtered and the obtained solid was washed with dichloromethane. The washing liquid and the filtrate were combined and the mixture was extracted with dichloromethane. The organic layer was washed with a 5% aqueous sodium hydroxide solution and the washing liquid was re-extracted with dichloromethane. This was combined with the organic layer and the mixture was washed with a saturated aqueous ammonium chloride solution and aqueous NaCl solution in this order, followed by drying with anhydrous magnesium sulfate and concentrating under reduced pressure to obtain 0.886 g of 3,6-dichloropyridazine-1-oxide (purity: 98.2%, yield: 78.5%).

### Example 5

11.52 ml (201 mmol) of 50% hydrogen peroxide was added to a mixture of 13.65 g (113.4 mmol) of anhydrous magnesium sulfate and 1,2-dichloroethane while cooling with an ice-cooled bath, and the mixture was stirred for 30 minutes. The ice-cooled bath was removed and 10.0 g (67.1 mmol) of 3,6-dichloropyridazine, 32.94 g (335.9 mmol) of maleic anhydride and 4.60 ml (67.1 mmol) of acrylic acid were added thereto, followed by stirring of the mixture at room temperature for 27 hours (the amount of water in the reaction system was 6.85 g (381 mmol), but it was almost completely dehydrated by the dehydrating agent (anhydrous magnesium sulfate)). An aqueous sodium hydroxide solution [prepared from 26.8 g (671 mmol) of sodium hydroxide and 150 ml of water] was added to the mixture under ice-cooling over 1 hour. Thereafter, 1,2-dichloroethane (100 ml) and 17.13 g (135.9 mmol) of sodium sulfite were added to the reaction mixture and the mixture was stirred under ice-cooling for 30 minutes. The mixture was filtered and the solid was washed with 1,2-dichloroethane. The filtrate and the washing liquid were combined and 50 ml of water was added thereto to separate the solution. The aqueous layer was extracted with 1,2-dichloroethane (2×50 ml) and the organic layer was combined with it. It was dried with anhydrous sodium sulfate, followed by concentrating under reduced pressure to obtain 10.00 g of 3,6-dichloropyridazine-1-oxide (purity: 98.5%, yield: 89.0%).

### Example 6

11.52 ml (201 mmol) of 50% hydrogen peroxide was added to a mixture of 10.0 g (67.1 mmol) of 3,6-dichloropyridazine, 59.3 g (605 mmol) of maleic anhydride, 0.58 g (6.7 mmol) of crotonic acid and 1,2-dichloroethane (100 ml) with stirring over 10 minutes. After the mixture was stirred at room temperature for 24 hours, 1,2-dichloroethane (100 ml) was added thereto and the mixture was ice-cooled (the amount of water in the reaction system was 6.85 g (381 mmol)). An aqueous sodium hydroxide solution [prepared from 48.3 g (1.21 mol) of sodium hydroxide and 200 ml of water] was added to the mixture under ice-cooling over 1 hour and 30 minutes. 2.6 g (20.6 mmol) of sodium sulfite was added thereto and the mixture was stirred under ice-cooling for 30 minutes. 50 ml of water was added thereto and the mixture was filtered. The filtrate was separated to obtain an organic layer A and an aqueous layer A. The aqueous layer A was extracted with 1,2-dichloroethane (3x50 ml) to obtain an organic layer B and an aqueous layer B. The organic layer A and the organic layer B were combined, followed by washing with water (100 ml) to obtain an organic layer C and an aqueous layer C. The aqueous layer C was extracted with 1,2-dichloroethane (2×50 ml) to obtain an organic layer D and an aqueous layer D. The organic layer C and the organic layer D were combined, followed by drying with anhydrous magnesium sulfate and concentrating under reduced pressure to obtain 9.73 g of 3,6-dichloropyridazine-1-oxide (purity: 93.2%, yield: 81.9%).

### Example 7

1.15 ml (20.1 mmol) of 50% hydrogen peroxide was added to a mixture of 1.0 g (6.71 mmol) of 3,6-dichloropyridazine, 3.31 g (33.8 mmol) of maleic anhydride, 0.0625 g (0.73 mmol) of crotonic acid and 1,2-dichloroethane (10 ml) with stirring. After the mixture was stirred at room temperature for 24 hours, 1,2-dichloroethane (10 ml) was added thereto and the mixture was ice-cooled (the amount of water in the reaction system was 0.685 g (38.1 mmol)). An aqueous sodium hydroxide solution [prepared from 2.72 g (68 mmol) of sodium hydroxide and 12 ml of water] was added to the mixture under ice-cooling over 20 minutes. 0.253 g (2.01 mmol) of sodium sulfite was added thereto and the mixture was stirred under ice-cooling for 10 minutes. The mixture was filtered and the filtrate was separated to obtain an organic layer A and an aqueous layer A. The aqueous layer A was extracted with 1,2-dichloroethane (3×5 ml) to obtain an organic layer B and an aqueous layer B. The organic layer A and the organic layer B were combined, followed by washing with water (10 ml) to obtain an organic layer C and an aqueous layer C. The aqueous layer C was extracted with 1,2-dichloroethane (2×5 ml) to obtain an organic layer D and an aqueous layer D. The organic layer C and the organic layer D were combined, followed by drying with anhydrous magnesium sulfate and concentrating under reduced pressure to obtain 0.9573 g of 3,6-dichloropyridazine-1-oxide (purity: 74.7%, yield: 64.6%).

### Example 8

0.129 ml (2.22 mmol) of 50% hydrogen peroxide was added to a mixture of 165.7 mg (1.11 mmol) of 3,6-dichloropyridazine, 499.8 mg (5.10 mmol) of maleic anhydride, 19.8 mg (0.223 mmol) of crotonic acid and heptane (2 ml) with stirring. After the mixture was stirred at 30°C for 22 hours, the mixture was left to stand for cooling and dichloromethane (10 ml) and a 10 w/v% aqueous sodium hydroxide solution (5 mL) were added thereto, followed by stirring of the mixture. The mixture was separated and the organic layer was concentrated to obtain 140.5 mg of 3,6-dichloropyridazine-1-oxide (purity: 97.8%, yield: 75.0%).

### Example 9

0.129 ml (2.22 mmol) of 50% hydrogen peroxide was added to a mixture of 165.3 mg (1.11 mmol) of 3,6-dichloropyridazine, 302.3 mg (3.08 mmol) of maleic anhydride, 20.5 mg (0.238 mmol) of crotonic acid and heptane (2 ml) with stirring. After the mixture was stirred at 65°C for 22 hours, the mixture was left to stand for cooling and dichloromethane (10 ml) and a 10 w/v% aqueous sodium hydroxide solution (3 mL) were added thereto, followed by stirring of the mixture. The mixture was separated and the organic layer was concentrated to obtain 118.0 mg of 3,6-dichloropyridazine-1-oxide (purity: 98.2%, yield: 63.3%).

### Example 10

0.097 ml (1.66 mmol) of 50% hydrogen peroxide was added to a mixture of 165.7 mg (1.11 mmol) of 3,6-dichloropyridazine, 300.8 mg (3.07 mmol) of maleic anhydride, 19.1 mg (0.222 mmol) of crotonic acid and heptane (2 ml) with stirring. After the mixture was stirred at 65°C for 22 hours and 30 minutes, the mixture was left to stand for cooling and dichloromethane (10 ml) and a 10 w/v% aqueous sodium hydroxide solution (3 mL) were added thereto, followed by stirring of the mixture. This was separated and the organic layer was concentrated to obtain 118.0 mg of 3,6-dichloropyridazine-1-oxide (purity: 92.4%, yield: 59.5%).

### Example 11

0.129 ml (2.22 mmol) of 50% hydrogen peroxide was added to a mixture of 165.1 mg (1.11 mmol) of 3,6-dichloropyridazine, 499.3 mg (5.09 mmol) of maleic anhydride, 19.1 mg (0.222 mmol) of crotonic acid and cyclohexane (2 ml) with stirring. After the mixture was stirred at 35°C for 22 hours, the mixture was left to stand for cooling and dichloromethane (10 ml) and a 10 w/v% aqueous sodium hydroxide solution (5 mL) were added thereto, followed by stirring of the mixture. The mixture was separated and the organic layer was concentrated to obtain 138.1 mg of 3,6-dichloropyridazine-1-oxide (purity: 97.8%, yield: 73.7%).

### Example 12

0.129 ml (2.22 mmol) of 50% hydrogen peroxide was added to a mixture of 165.3 mg (1.11 mmol) of 3,6-dichloropyridazine, 499.5 mg (5.09 mmol) of maleic anhydride, 19.3 mg (0.224 mmol) of crotonic acid and methylcyclohexane (2 ml) with stirring. After the mixture was stirred at 35°C for 22 hours, the mixture was left to stand for cooling and dichloromethane (10 ml) and a 10 w/v% aqueous sodium hydroxide solution (5 mL) were added thereto, followed by stirring of the mixture. The mixture was separated and the organic layer was concentrated to obtain 138.6 mg of 3,6-dichloropyridazine-1-oxide (purity: 98.2%, yield: 74.3%).

### Example 13

0.244 ml (4.19 mmol) of 50% hydrogen peroxide was added to a mixture of 166.4 mg (1.12 mmol) of 3,6-dichloropyridazine, 500.1 mg (5.10 mmol) of maleic anhydride, 21.0 mg (0.244 mmol) of crotonic acid and chlorobenzene (2 ml) with stirring. After the mixture was stirred at room temperature for 22 hours, dichloromethane (10 ml) and a 10 w/v% aqueous sodium hydroxide solution (5 mL) were added thereto, followed by stirring of the mixture. The mixture was separated and the organic layer was concentrated to obtain 143.4 mg of 3,6-dichloropyridazine-1-oxide (purity: 82.2%, yield: 63.8%).

### Example 14

0.244 ml (4.19 mmol) of 50% hydrogen peroxide was added to a mixture of 165.4 mg (1.11 mmol) of 3,6-dichloropyridazine, 500.1 mg (5.10 mmol) of maleic anhydride, 21.0 mg (0.244 mmol) of crotonic acid and 2-chlorotoluene (2 ml) with stirring. After the mixture was stirred at room temperature for 22 hours, dichloromethane (10 ml) and a 10 w/v% aqueous sodium hydroxide solution (5 mL) were added thereto, followed by stirring of the mixture. The mixture was separated and the organic layer was concentrated to obtain 138.5 mg of 3,6-dichloropyridazine-1-oxide (purity: 87.7%, yield: 66.3%).

### Example 15

0.345 ml (3.32 mmol) of 30% hydrogen peroxide was added to a mixture of 165.3 mg (1.11 mmol) of 3,6-dichloropyridazine, 1000.2 mg (10.2 mmol) of maleic anhydride, 19.2 mg (0.223 mmol) of crotonic acid and 1,2-dichloroethane (2 ml) with stirring. After the mixture was stirred at 30°C for 22 hours, the mixture was left to stand for cooling and dichloromethane (10 ml) and a 10 w/v% aqueous sodium hydroxide solution (10 mL) were added thereto, followed by stirring of the mixture. The mixture was separated and the organic layer was concentrated to obtain 144.8 mg of 3,6-dichloropyridazine-1-oxide (purity: 83.4%, yield: 65.9%).

### Example 16

0.345 ml (3.32 mmol) of 30% hydrogen peroxide was added to a mixture of 165.5 mg (1.11 mmol) of 3,6-dichloropyridazine, 1001.0 mg (10.2 mmol) of maleic anhydride, 19.2 mg (0.223 mmol) of crotonic acid and heptane (2 ml) with stirring. After the mixture was stirred at 34°C for 22 hours, the mixture was left to stand for cooling and dichloromethane (10 ml) and a 10 w/v% aqueous sodium hydroxide solution (10 mL) were added thereto, followed by stirring of the mixture. The mixture was separated and the organic layer was concentrated to obtain 132.4 mg of 3,6-dichloropyridazine-1-oxide (purity: 98.7%, yield: 71.3%).

### Example 17

1.32 g (14.0 mmol) of urea hydrogen peroxide addition compound was suspended in 10 mL of dichloromethane, and 1.69 mL (12.0 mmol) of trifluoroacetic anhydride was added thereto under ice-cooling. After the mixture was stirred at room temperature for 30 minutes, it was ice-cooled again and 1.43 g (9.60 mmol) of 3,6-dichloropyridazine was added thereto, followed by stirring of the mixture at room temperature for 12 hours. A 10% aqueous sodium sulfite solution was added to the reaction mixture and excessive urea hydrogen peroxide addition compound was decomposed and extracted with ethyl acetate. The organic layer was washed with a saturated aqueous sodium hydrogencarbonate solution, water and an aqueous NaCl solution in this order, dried with anhydrous magnesium sulfate and concentrated under reduced pressure to obtain 1.47 g (yield: 93%) of 3,6-dichloropyridazine-1-oxide.
¹H-NMR(500MHz,CDCl₃) δ (ppm): 7.79 (1H, d, J = 8.2 Hz), 7.13 (1H, d, J = 8.2 Hz).

### Example 18

1.01 g (6.78 mmol) of 3,6-dichloropyridazine and 2.26 g (13.5 mmol) of dichloromaleic anhydride were suspended in 10 mL of 1,2-dichloroethane, and 0.26 mL (3.4 mmol) of trifluoroacetic acid was added thereto. Subsequently, 1.27 g (13.5 mmol) of urea hydrogen peroxide addition compound was added thereto and the mixture was stirred at room temperature for 18 hours. A 10% aqueous sodium sulfite solution (10 mL) was added to the reaction mixture and after the mixture was stirred at 0°C for 1 hour, the mixture was extracted with dichloromethane. The organic layer was washed with a 4% aqueous sodium hydroxide solution and the washing liquid was re-extracted with dichloromethane. The extract was combined with the organic layer, followed by washing with a saturated aqueous ammonium chloride solution and aqueous NaCl solution in this order, drying with anhydrous magnesium sulfate and concentrating under reduced pressure to obtain 980.4 mg (purity: 99.6%) of 3,6-dichloropyridazine-1-oxide. Accordingly, the net yield of 3,6-dichloropyridazine-1-oxide was 976 mg (yield: 87%).

### Example 19

1.01 g (6.78 mmol) of 3,6-dichloropyridazine and 2.25 g (13.5 mmol) of dichloromaleic anhydride were suspended in 8 mL of 1,2-dichloroethane, and 2.4 mL (42 mmol) of acetic acid was added thereto. Subsequently, 1.26 g (13.4 mmol) of urea hydrogen peroxide addition compound was added thereto and the mixture was stirred at room temperature for 26 hours. The reaction liquid was concentrated and dichloromethane and a 10% aqueous sodium sulfite solution (10 mL) were added to the residue. After the mixture was stirred at 0°C for 1 hour, it was extracted with dichloromethane. The organic layer was washed with a 4% aqueous sodium hydroxide solution and the washing liquid was re-extracted with dichloromethane. The extract was combined with the organic layer and it was washed with a saturated aqueous ammonium chloride solution and aqueous NaCl solution in this order, followed by drying with anhydrous magnesium sulfate and concentrating under reduced pressure to obtain 935.2 mg (purity: 99.0%) of 3,6-dichloropyridazine-1-oxide. Accordingly, the net yield of 3,6-dichloropyridazine-1-oxide was 926 mg (yield: 83%).

### Example 20

10.0 g (67.1 mmol) of 3,6-dichloropyridazine and 22.3 g (134 mmol) of dichloromaleic anhydride were suspended in 100 mL of 1,2-dichloroethane and the suspension was stirred in a water bath. 12.6 g (134 mmol) of urea hydrogen peroxide addition compound was added thereto over 1.5 hours while dividing the compound into four portions. The mixture was stirred for 17 hours and a 10% aqueous sodium sulfite solution (100 mL) was added to the reaction mixture. After the mixture was stirred at 0°C for 2.5 hours, it was extracted with dichloromethane. The organic layer was washed with a 4% aqueous sodium hydroxide solution and the washing liquid was re-extracted with dichloromethane. The extract was combined with the organic layer and the mixture was washed with a saturated aqueous ammonium chloride solution and aqueous NaCl solution in this order, followed by drying with anhydrous magnesium sulfate and concentrating under reduced pressure to obtain 9.80 g (purity: 96.2%) of 3,6-dichloropyridazine-1-oxide. Accordingly, the net yield of 3,6-dichloropyridazine-1-oxide was 9.43 g (yield: 85%).

### Example 21

578 mg (2.02 mmol) of tetrachlorophthalic anhydride, 191 mg (2.03 mmol) of urea hydrogen peroxide addition compound and 151 mg (1.01 mmol) of 3,6-dichloropyridazine were suspended in 2 mL of acetic acid and the suspension was stirred at room temperature for 2 days. When a part of the reaction mixture was sampled and measured by ¹H-NMR, the peaks other than those of 3,6-dichloropyridazine and 3,6-dichloropyridazine-1-oxide were not observed. Further, the production ratio was found to be 1:4 in a molar ratio. This meant that a conversion ratio of reaction was 80%.

### Example 22

1.01 g (6.78 mmol) of 3,6-dichloropyridazine and 1.33 g (13.6 mmol) of maleic anhydride were dissolved in a solvent mixture of 5 mL dichloromethane and 5 mL acetic acid, and 1.27 g (13.5 mmol) of urea hydrogen peroxide addition compound was added thereto at room temperature. The mixture was stirred for 3 days and after the reaction mixture was concentrated, dichloromethane and a 10% aqueous sodium sulfite solution (10 mL) were added thereto and the mixture was stirred at 0°C for 0.5 hours, followed by extraction of the mixture with dichloromethane. The organic layer was washed with a 4% aqueous sodium hydroxide solution and the washing liquid was re-extracted with dichloromethane. The extract was combined with the organic layer and the mixture was washed with a saturated aqueous ammonium chloride solution and aqueous NaCl solution in this order, followed by drying with anhydrous magnesium sulfate and concentrating under reduced pressure to obtain 774.6 mg (purity: 88.6%) of 3,6-dichloropyridazine-1-oxide. Accordingly, the net yield of 3,6-dichloropyridazine-1-oxide was 686 mg (yield: 61%).

### Reference example

### Reference example 1

### 6-Chloro-3-(2,6-dimethylphenoxy)-4-pyridazinol

### (1) 6-Chloro-3-(2,6-dimethylphenoxy)pyridazine 1-oxide

268 mg (2.20 mmol) of 2,6-dimethylphenol, 1,4-dioxane (3 mL) and dimethyl sulfoxide (3 mL) were mixed, and 270 mg (2.41 mmol) of potassium tert-butoxide was added to the mixture under ice-cooling, followed by stirring of the mixture for 10 minutes. 370 mg (2.24 mmol) of 3,6-dichloropyridazine 1-oxide prepared by the process of the above Example 3 or 19 was added thereto and after the mixture was stirred at room temperature for 10 hours, it was left to stand for 2 days. The reaction mixture was poured to ice-cooled water and extracted with ethyl acetate. The organic layers were combined and successively washed with water and a saturated aqueous NaCl solution, followed by drying with anhydrous sodium sulfate. The solvent was distilled off and the residue was purified by silica gel column chromatography (hexane:ethyl acetate, gradient) to obtain 350 mg (1.39 mmol, yield: 63.1%) of 6-chloro-3-(2,6-dimethylphenoxy)pyridazine 1-oxide.

### (2) 4,6-Dichloro-3-(2,6-dimethylphenoxy)pyridazine

330 mg (1.31 mmol) of 6-chloro-3-(2,6-dimethylphenoxy)pyridazine 1-oxide obtained by (1) was mixed with dichloromethane (0.6 mL) and 0.60 mL (6.5 mmol) of phosphorus oxychloride were mixed, and the mixture was stirred for 1 hour and further left to stand for 5 days. The reaction mixture was poured to ice-cooled water and extracted with ethyl acetate. The organic layers were combined and successively washed with water and a saturated aqueous NaCl solution, followed by drying with anhydrous sodium sulfate. The solvent was distilled off and the residue was purified by silica gel column chromatography (hexane:ethyl acetate, gradient) to obtain 322 mg (1.20 mmol, yield: 91.6%) of 4,6-dichloro-3-(2,6-dimethylphenoxy)pyridazine.

### (3) 6-Chloro-3-(2,6-dimethylphenoxy)-4-pyridazinol

300 mg (1.12 mmol) of 4,6-dichloro-3-(2,6-dimethylphenoxy)pyridazine obtained by (2) was dissolved in dimethyl sulfoxide (8 mL), and 0.80 mL (2.0 mmol) of a 10% (W/V) aqueous sodium hydroxide solution was added to the solution, followed by stirring of the mixture at room temperature overnight. Further, 0.80 mL (2.0 mmol) of a 10% (W/V) aqueous sodium hydroxide solution was added and after disappearing the raw material, the reaction mixture was poured to ice-cooled water. After the mixture was acidified with hydrochloric acid, it was extracted with ethyl acetate. The organic layers were combined and successively washed with water and a saturated aqueous NaCl solution, followed by drying with anhydrous sodium sulfate. The solvent was distilled off and the residue was purified by silica gel column chromatography (hexane:ethyl acetate, gradient) and preparative thin layer chromatography (manufactured by Merck Inc., 1.05744, developed by dichloromethane:methanol=9:1) to obtain 128 mg (0.510 mmol, yield: 45.5%) of 6-chloro-3-(2,6-dimethylphenoxy)-4-pyridazinol.
¹H-NMR (200MHz, DMSO-d₆) δ ppm: 7.18-7.05 (3H, m), 6.83 (1H, s), 2.05 (6H, s).
Melting point (°C):214-215.

The compound synthesized in Reference Example 1 has herbicidal action, and can be used as a herbicide. In rice paddies, for example, the compound synthesized in Reference Example 1 demonstrates herbicidal activity against dominant weeds of rice paddies, e.g. annual broadleaf weeds such as *Lindernia* spp. and *Rotala indica,* and perennial weeds of the Cyperaceae family such as *Scirpus joncoides* and *Cyperus serotinus,* and weeds of the Graminaceous family such as *Echinochloa oryzicola,* without demonstrating problems in terms of chemical toxicity with respect to rice, by a flooding soil treatment before or after weed germination.

### Reference Example 2

### (Wettable Powder)

The compound obtained in Reference Example 1 (10 parts by mass), Carplex #80D (Shionogi & Co., Ltd., 10 parts by mass), Gohsenol GL05 (Nippon Synthetic Chemical Industry Co., Ltd., 2 parts by mass), Newcol 291PG (dioctylsulfosuccinate sodium salt, Nippon Nyukazai Co., Ltd., 0.5 parts by mass) Neogen Powder (Dai-ichi Kogyo Seiyaku Co., Ltd., 5 parts by mass), Radiolite #200 (Showa Chemical Industry Co., Ltd., 10 parts by mass) and H Bibun (Keiwa Rozai Co., Ltd., 62.5 parts by mass) were mixed well followed by crushing with an Ecksample Model KII-1 (Fuji Paudal Co., Ltd.) to obtain a wettable powder.

### Test Examples

The following lists biological test examples and indicates specific effects.

### Test Example 1

### Treatment Prior to Germination of Rice Paddy Weeds

Rice paddy soil was filled into a 1/10,000a pot, followed by mixing seeds of dormancy awakening *Echinochloa oryzicola* and *Scirpus joncoides,* and annual broadleaf weeds (*Lindernia* spp. and *Rotala indica*) in 1 cm of the surface layer of soil. In addition, *Cyperus serotinus* tubers subjected to accelerated germination were planted, followed by transplanting rice seedlings at the 2.2 leaf stage and growing under flooding conditions in a greenhouse. Three days after transplant, a predetermined dose of the wettable powder prepared according to Reference Example 2 was diluted with water, the soil was treated with a spraying solution thereof under flooding conditions and herbicidal effects and chemical damage to the transplanted rice plants were measured according to the evaluation criteria shown below 25 days after treatment.

### (Evaluation Criteria)

0: Growth inhibition rate 0 to 10%
1: Growth inhibition rate 11 to 30%
2: Growth inhibition rate 31 to 50%
3: Growth inhibition rate 51 to 70%
4: Growth inhibition rate 71 to 90%
5: Growth inhibition rate 91 to 100%

As a result, the compound synthesized in Reference Example 1 demonstrated activity evaluated as 5 against broadleaf weeds, *Scirpus joncoides* and *Cyperus serotinus* and demonstrated activity evaluated as 2 against *Echinochloa oryzicola* at a dose of 10 g/a. On the other hand, activity against rice plants was evaluated as 0 (no chemical damage).

### INDUSTRIAL APPLICABILITY

Since the present invention is able to provide 3,6-dichloropyridazine-1-oxide easily and at high yield, various physiologically active substances can be advantageously synthesized by using it as a synthesis intermediate.

## Claims

1. A process for preparing 3,6-dichloropyridazine-1-oxide which comprises reacting 3,6-dichloropyridazine with an acid anhydride and hydrogen peroxide of a concentration of 60% or less or a urea hydrogen peroxide addition compound.

2. The preparation process according to Claim 1, wherein 3,6-dichloropyridazine is reacted with the acid anhydride and the hydrogen peroxide of a concentration of 60% or less.

3. The preparation process according to Claim 1, wherein 3,6-dichloropyridazine is reacted with the acid anhydride and the urea hydrogen peroxide addition compound.

4. The preparation process according to any one of Claims 1 to 3, wherein the acid anhydride is one kind or more of acid anhydrides selected from acetic/formic anhydride, acetic anhydride, trichloroacetic anhydride, trifluoroacetic anhydride, propionic anhydride, butyric anhydride, succinic anhydride, maleic anhydride, dichloromaleic anhydride, phthalic anhydride, 3,6-dichlorophthalic anhydride, tetrachlorophthalic anhydride and tetrabromophthalic anhydride.

5. The preparation process according to Claim 4, wherein the acid anhydride is one kind or more of acid anhydrides selected from trifluoroacetic anhydride, maleic anhydride, dichloromaleic anhydride, 3,6-dichlorophthalic anhydride, tetrachlorophthalic anhydride and tetrabromophthalic anhydride.

6. The preparation process according to Claim 5, wherein the acid anhydride is maleic anhydride.

7. The preparation process according to Claim 5, wherein the acid anhydride is dichloromaleic anhydride.

8. The preparation process according to Claim 5, wherein the acid anhydride is trifluoroacetic anhydride.

9. The preparation process according to Claim 5, wherein the acid anhydride is tetrachlorophthalic anhydride.

10. The preparation process according to any one of Claims 1 to 9, wherein an acid is added to the reaction system.

11. The preparation process according to Claim 10, wherein the acid is formic acid, acetic acid, propionic acid, butyric acid, isobutyric acid, pivalic acid, cyclohexanecarboxylic acid, 1-methylcyclohexanecarboxylic acid, 1-adamantanecarboxylic acid, acrylic acid, methacrylic acid, crotonic acid, 3,3-dimethylacrylic acid, tiglic acid, cinnamic acid, trifluoroacetic acid, chloroacetic acid, benzoic acid, methanesulfonic acid, trifluoromethanesulfonic acid or p-toluenesulfonic acid.

12. The preparation process according to Claim 11, wherein the acid is formic acid, acetic acid, propionic acid, butyric acid, isobutyric acid, pivalic acid, cyclohexanecarboxylic acid, 1-methylcyclohexanecarboxylic acid, 1-adamantanecarboxylic acid, acrylic acid, methacrylic acid, crotonic acid, 3,3-dimethylacrylic acid, tiglic acid, cinnamic acid, trifluoroacetic acid or chloroacetic acid.

13. The preparation process according to any one of Claims 1 to 12, wherein a dehydrating agent is added to the reaction system.

14. The preparation process according to Claim 13, wherein the dehydrating agent is anhydrous magnesium sulfate.

15. The preparation process according to any one of Claims 1 to 14, wherein the hydrogen peroxide is added in an amount of 1 to 5 equivalents and the acid anhydride is added in an amount of excessive mole number relative to the mole number of water existing in the reaction system, or the urea hydrogen peroxide addition compound is added in an amount of 1 to 5 equivalents, with respect to 3,6-dichloropyridazine.
